# EUROPÄISCHE PATENTSCHRIFT

(11) **EP 1 166 804 B1**
(45) Veröffentlichungstag und Bekanntmachung des Hinweises auf die Patenterteilung: **06.08.2003**
(21) Anmeldenummer: 01112667.9
(22) Anmeldetag: 25.05.2001
(51) Int. Cl.: A61L 27/32, A61L 27/34

(54) **Knochenanaloge Beschichtung für metallische Implantatmaterialien**
Bone-like coating for metallic implant materials
Revêtement ressemblant à l'os naturel pour implants métalliques

(30) Priorität: 21.06.2000 DE 10029520
(43) Veröffentlichungstag der Anmeldung: 02.01.2002
(73) Patentinhaber: MERCK PATENT GmbH, 64293 Darmstadt (DE)
(72) Erfinder: Sewing, Andreas, 64807 Dieburg (DE); Dard, Michel, 64342 Seeheim-Jugenheim (DE); Rössler, Sophie, 01309 Dresden (DE); Scharnweber, Dieter, 01324 Dresden (DE); Worch, Hartmut, 01069 Dresden (DE)

(56) Entgegenhaltungen:
- EP-A- 0 389 713
- DE-A- 19 811 900
- US-A- 5 508 267
- SHIRKHANZADEH: "Direct formation of nanophase hydroxyapatite on cathodically polarized electrodes" JOURNAL OF MATERIALS SCIENCE: MATERIALS IN MEDICINE, Bd. 9, 1998, Seiten 67-72, XP002182795

## Beschreibung

Die Erfindung betrifft eine biomimetisch erzeugte knochenanaloge Beschichtung bestehend aus einem organischen und anorganischen Hauptbestandteil für metallische Implantatmaterialien beliebiger Oberflächengeometrie sowie ein Verfahren zu deren Herstellung. Wesentliche Komponenten dieser Beschichtung sind Kollagen und Calciumphosphatphasen, die den organischen und anorganischen Hauptbestandteil des Knochens bilden. Die erfindungsgemäße Beschichtung eignet sich im besonderen Maße als Matrix zur Einbindung weiterer induktiver Substanzen wie z.B. Wachstumsfaktoren, Adhäsionsproteine oder pharmakologische Wirkstoffe.

Unter der Fragestellung einer verbesserten Anpassung der physikalischchemischen und biochemischen Eigenschaften der Oberflächen von Implantaten an das lokale Umgebungsgewebe mit dem Ziel der Optimierung der Biokompatibilität und -funktionalität werden verschiedene Ansätze verfolgt.
Neben reinen Veränderungen der Topographie der Implantatoberfläche, wie z. B. Ätzen oder Sandstrahlen, spielen momentan Beschichtungen mit Calciumphosphatphasen (CPP) eine bedeutende Rolle. In der Anwendung am weitesten fortgeschritten ist die Beschichtung von Implantaten im Knochenkontakt mit Hydroxylapatit und zunehmend auch leichter löslichen Calciumphosphatphasen [Yang et al, J. Mater. Sci,: Mater. in Med. **6,** 258-65 (1995); Remer, P. Schwerpunktprogramm Gradientenwerkstoffe, 3. Ausg. Darmstadt 31.3.1998; Floquet et al., Rev. Stomatol. Chir. Maxillofac. 98, 47-9 (1997)]. Diese Methoden der Beschichtung von Implantaten mit der anorganischen Hauptkomponente von Knochen und davon abgeleiteten Verbindungen zielen besonders auf ein schnelleres Einwachsen des Implantats durch ein lokal erhöhtes Angebot an Calciumund Phosphationen. Die Beschichtung von Implantatoberflächen mit CPP erfolgt zur Zeit überwiegend durch Plasmaspritzverfahren. Aufgrund der Prozeßbedingungen weisen diese Schichten Eigenschaften auf, die in Kristallinität und Lösungsverhalten von der mineralischen Phase des Knochens stark abweichen und aufgrund der hohen Schichtdicken zum mechanischen Versagen der Schichten führen können [Filiaggi et al., J. Biomed Mat. Res. **27**(2), 191-8 (1993); Gross et al., Int. J. Oral Maxillofac. Implants **12** (5), 589-97 (1997); Posner et al., Phosphate Minerals, Springer Verlag, Berlin/Heidelberg (1984)].

Elektrochemisch gestützte Verfahren [Shirkhanzadeh, J. Mater. Sci.:Mater. in Med. 9, 76-72 (1998); Szmukler-Moncler et al., Biological Mech. Of Tooth Eruption, Resorption and Replacement by implants, (Eds. Z. Davidovitch and J. Mah), 481-85 Harvard Society for the Advancement of Orthodontics, Boston, USA (1998)] bieten die Möglichkeit, CPP mit geringeren Schichtdicken zu erzeugen. Die Abscheidung von CPP wird durch kathodische Polarisation des Implantats in Ca²⁺/HₓPO₄^{(3-x)-}-haltiger Lösung realisiert. Die Polarisation des Implantats führt zu einer Alkalisierung des oberflächennahen Elektrolyten (2H₂O + 2e⁻ → H₂ + 2OH⁻), wodurch vor der Probenoberfläche eine Fällungs-Reaktion ausgelöst und das gebildete Fällungsprodukt auf der metallischen Implantatoberfläche abgeschieden wird.

Ein weiterer Ansatz auf dem Gebiet der Oberflächenmodifizierung von Implantatwerkstoffen besteht darin, eine Biologisierung von Implantatoberflächen zu erreichen, indem im Umgebungsgewebe vorkommende organische Verbindungen für die Oberflächenmodifizierung genutzt werden. Hierbei werden zum einen immobilisierte Proteine und Proteinsequenzen verwendet, die ihre Wirkung im immobilisierten Status ausüben (Kollagen, Adhäsionsproteine, RGD-Sequenzen) bzw. Proteine die über einen bestimmten Zeitraum freigesetzt werden. Je nach immobilisierter Substanz wird dabei eine weitgehend allgemeine, positive Wirkung auf die Biokompatibilität der Implantatoberfläche (Kollagen, bestimmte Adhäsionsproteine) oder die Adhäsion bestimmter Zelltypen angestrebt (erweiterte RGD-Sequenzen)[Schaffner et al., J. of Mat. Sci.: Mat. in Med. **10,** 837-39 (1999)]

Der vorangehend genannte Stand der Technik zeigt, dass Verfahren, die sich die Erzeugung einer knochenanalogen Verbundphase aus den mineralischen und organischen Bestandteilen des Knochens für die Beschichtung metallischer Implantate zum Ziel gesetzt haben, bisher nicht bekannt sind. Methoden, die sowohl Hydroxylapatit als auch Kollagen beinhalten, beschränken sich lediglich auf Mischungen der Komponenten, die zudem auf weitere körperfremde Stoffe als Trägermaterialien angewiesen sind.

Die WO 99/30672 (Uni Tübingen) beschreibt eine Beschichtung für Prothesen aus organischen Polymermaterial in deren Oberfläche Hydroxylapatit oder Kollagen eingebunden werden kann. Das Polymermaterial stellt hier lediglich den Haftvermittler dar, von einem knochenähnlichen Verbund von Kollagen und einer Calciumphosphatphase kann nicht gesprochen werden.

Eine weitere Möglichkeit der Einbindung von Skleroproteinen und Calciumphosphat wird in DE19811900 (Feinchemie) dargestellt. Ein biokompatibles Kompositmaterial, bestehend aus einem anorganischen Gel und einer bioaktiven Komponente (Kollagen, Elastin, Fibrin), wird beschrieben. Außerdem können Calciumphosphate oder deren Vorstufen in gelöster Form enthalten sein. Dieses Kompositmaterial stellt demnach nur eine Mischung der Hauptbestandteile des Knochens dar, die zudem auf ein anorganisches Gel als Träger angewiesen ist.
In WO 92/13984 (Queen's University of Kingston) wird ein Verfahren zur elektrochemischen Erzeugung keramischer Beschichtungen aus Calciumphosphatverbindungen beschrieben. Hierbei wird nicht ausgeschlossen, daß der Elektrolyt auch biologische nicht toxische Verbindungen wie Kollagen oder Verunreinigungen enthält. Die Beschichtung stellt ein einheitliches microporöses keramisches Material aus verbundenen nicht orientierten Kristalliten dar. Diese Schicht kann als Ausfällungsprodukte auch biologisch aktive Verbindungen enthalten. Die beschriebene Beschichtung unterscheidet sich als keramische Calciumphosphatbeschichtung demnach deutlich von einer mineralisierten Kollagen-Calciumphosphat-Matrix.

Implantate für den Einsatz im Kieferbereich oder Gelenkersatz werden vorzugsweise aus metallischen Werkstoffen gefertigt, um den mechanischen Anforderungen gerecht zu werden. Dabei wird die unmittelbare Oberfläche, die sich in ihren Eigenschaften stark von dem Grundwerkstoff unterscheiden kann, oft vernachlässigt. Es ist jedoch bekannt, daß gerade die Eigenschaften der Oberfläche für die Wechselwirkungen zwischen Implantat und Umgebungsgewebe von entscheidender Bedeutung sind. So können Konformationsänderungen von adsorbierten Proteinen wesentlich zur Bildung einer fibrösen Zwischenschicht beitragen, die wiederum in einer unzureichenden Stabilität des Implantats resultieren kann.

Die Lehre der vorliegenden Erfindung geht von der Aufgabe aus, Implantatoberflächen gezielt mit biochemischen Informationen zu modifizieren, um nach der Implantation eine rasche Osteointegration unter Bildung hochwertigen Knochengewebes zu erreichen.

Diese Aufgabe wird durch eine knochenanaloge Beschichtung bestehend aus einem organischen und anorganischen Hauptbestandteil für metallische Implantatmaterialien beliebiger Oberflächengeometrie gelöst, wobei die Beschichtung im wesentlichen aus einer mit Calciumphosphat mineralisierter Kollagenmatrix besteht.

Als metallische Implantatmaterialien kommen generell alle in der Dentaltechnik oder in den Bereichen Endoprothetik und Trauma verwendeten Metalle in Frage. Besonders bevorzugt sind Titan und Titanlegierungen wie z.B. TiAl6V4.

Die erfindungsgemäße Beschichtung wird unter Bedingungen erzeugt, die das Einbeziehen von organischen Komponenten möglich macht. Die Erfindung zur biomimetischen Erzeugung einer knochenanalogen Matrix nutzt deshalb elektrochemisch gestützte Prozesse, die bei nahezu physiologischen pH- und Temperaturbedingungen durchgeführt werden können und somit das Einbeziehen von Biomolekülen möglich machen.
Diese können in der Elektrolytlösung oder in immobilisierter Form an der Implantatoberfläche vorliegen. Die Hauptkomponenten der Schicht bestehen aus Kollagen und Hydroxylapatit, der organischen bzw. anorganischen Hauptkomponente des Knochens. Durch den erfindungsgemäßen Gegenstand ist es erstmalig möglich, ein Durchdringungsgefüge analog der *in vivo* erzeugten Knochenstruktur in den wesentlichen Grundzügen *in vitro* nachzuvollziehen und mit guter Haftfestigkeit auf einer metallischen Implantatoberfläche zu erzeugen. Der Aufbau der mineralisierten Kollagenmatrix erfolgt schichtförmig. Dies hat den Vorteil, dass hierdurch auch die Erzeugung gradierter Schichten mit variierendem Mineralisierungsgrad der Kollagenmatrix möglich ist.

Der anorganische Hauptbestandteil bzw. die Calciumphosphatphase (CPP) besteht bevorzugt aus amorphen Calciumphosphat (Ca₉(PO₄)₆ • nH₂O), Hydroxylapatit (Ca₁₀(PO₄)₆(OH)₂), Octacalciumphosphat (Ca₈H₂(PO₄)₆• H₂O) oder Brushit (CaHPO₄• 2H₂O). Es sind aber auch Mischungen der vorher genannten Phasen möglich.

Die Calciumphosphatphase kann zusätzlich mit Ionen wie Fluorid, Silber, Magnesium oder Carbonat dotiert sein.

Bevorzugt ist die Verwendung von Typ I-Kollagen, welches im Knochen für die elastischen Eigenschaften verantwortlich ist und im mineralisierten Zustand zusammen mit den Hydroxylapatitkristalliten die hohe Festigkeit des Knochens bewirkt. Ferner kann das Kollagen auch aus einer Mischung der Typen I bis III bestehen. Die Typen I bis III gehören zu der Gruppe der Fibrillen-bildenden Kollagene. Zusätzlich kann dem Kollagen Gelatine zugesetzt werden. Neben Kollagen, das auch aus rekombinanter Produktion stammen kann, ist auch die Einbeziehung anderer Matrixproteine möglich.

Ein weiterer Vorteil der Erfindung besteht in der Möglichkeit, die beschriebenen Schichten als Matix für knochenspezifische Proteine (BMP, TGFβ etc.) zu nutzen. Neben Wachstumsfaktoren und zellspezifischen Adhäsionspeptiden ist auch die Einbindung pharmakologischer Wirkstoffe, wie z.B. Antibiotika, möglich.
Gegenstand der Erfindung ist ferner ein metallisches Implantat aus einem Grundkörper und einer von diesem getragenen Außenschicht, wobei die Außenschicht aus der erfindungsgemäßen Beschichtung nach einen der Ansprüche 1 bis 8 besteht.

Gegenstand der Erfindung ist auch ein Verfahren zur elektrochemisch gestützten Beschichtung von metallischen Implantatmaterialien beliebiger Oberfläche mit Kollagen und CPP gemäß Anspruch 11.

Die Beschichtung wird in einer Elektrolysezelle durchgeführt, in der das metallische Implantat kathodisch polarisiert wird. Die Schichtabscheidung erfolgt nahe physiologischen pH- und Temperaturbedingungen. Der Elektrolyt besteht aus einer Ca²⁺/HₓPO₄^{(3-x)-}-haltigen Lösung, die zusätzlich Kollagen bzw. andere Substanzen (Wachstumsfaktoren, Antibiotika) enthalten kann. Die Implantatoberfläche kann jede beliebige Oberflächengeometrie (Struktur; rauh, poliert, geätzt), eine chemische Modifikation (Erzeugung funktionaler Gruppen), eine Calciumphosphatschicht, eine Proteinschicht sowie eine Schicht hergestellt nach Patent Nr. WO 98/17844 (TU Dresden) oder DE-19504386 (TU Dresden) oder eine Kombination derselben aufweisen. Durch einen simultan geführten Prozeß der Calciumphosphatabscheidung und der Immobilisierung von Kollagen unter physiologischen pH- und Temperaturbedingungen kann eine mineralisierte Kollagenschicht auf der Titanoberfläche erzeugt werden. Der Grad der Mineralisierung, d.h. Art der CPP und Bedeckungsgrad werden dabei durch die elektrochemischen Parameter vorgegeben. Unterstützt werden kann dieser Prozeß durch die Zugabe von die Mineralisierung beeinflussenden Stoffgruppen (z. B. Bonesialoprotein, Osteopontin).

Ferner bevorzugt ist ein Beschichtungsverfahren gemäß Anspruch 12, wobei zunächst eine Beschichtung der Probe mit CPP in einem elektrochemischen Prozess über galvanostatische Polarisation in einer Calciumionen und Phosphationen enthaltenen Elektrolytlösung bei genau definierter Stromdichte und Temperatur, gefolgt von einer Beschichtung der mit CPP-beschichteten Probe durch Eintauchen in eine Kollagenlösung bei einem pH-Wert kleiner 8 und einer Temperatur zwischen 4 und 40°C für wenige Minuten erfolgt und anschließender Beschichtung der Kollagen-CPP-beschichteten Probe mit weiterem CPP in einem erneutem elektrochemischen Prozess über galvanostatische Polarisation unter genau definierter Stromdichte und Temperatur.

Die vorher genannten Verfahrensschritte können bevorzugt auch mehrfach alternierend ablaufen, d.h. eine Abfolge der Verfahrensschritte a) und b) gemäß Anspruch 11 nach dem Schema a-b-a-b-a-b usw.

Bevorzugt ist auch ein Verfahren gemäß Anspruch 14, wobei die Verfahrensschritte a) und b) in einem Schritt zusammengefaßt werden, wobei das zu beschichtende metallische Implantatmaterial in einer Calciumionen und Phosphationen enthaltenen Kollagenlösung kathodisch elektrochemisch polarisiert wird.

Noch bevorzugter ist ein Verfahren, in dem während der galvanostatischen Polarisation im Verfahrensschritt b) ein kathodischer Stromfluss von -0.5 bis -30 mA/cm² etwa 30 Minuten lang fließt.

Die Vorzüge der erfindungsgemäßen knochenanalogen mineralisierten Kollagenmatrix lassen sich eindrucksvoll im Zellversuch zeigen. Während die Zelladhäsion für Osteoblasten nach einer Stunde noch vergleichbar gute Werte mit biomimetisch erzeugten Hydroxylapatit-Schichten zeigt, ist die Zellproliferation auf den erfindungsgemäßen Schichten deutlich bevorzugt. Der Anstieg der Zellzahl erfolgt hier zu einem wesentlich früherem Zeitpunkt und der Maximalwert der Zellzahl wird sehr viel schneller erreicht als für reine Hydroxylapatit-Schichten. Eine entsprechende Meßkurve für einen Proliferationsversuch über 17 Tage mit MC3T3 Maus-Osteoblaten zeigt Abbildung 1.

Die Erfindung wird nachfolgend anhand von Ausführungsbeispielen unter Bezugnahme auf die Abbildung 1 näher beschrieben und erläutert.

### Beispiel 1

Ein Zylinder aus TiAl6V4 (h = 2 mm, ⌀ 10 mm) wird metallographisch mit einer abschließenden TiO₂-Politur präpariert. Der Zylinder wird anschließend in Aceton und Ethanol im Ultraschallbad gereinigt und mit destilliertem Wasser gespült.
Die Probe wird anschließend in eine Kollagenlösung getaucht, die folgendermaßen hergestellt wird: Säurelösliches gefriergetrocknetes Kalbshautkollagen Typ I wird in 0,01M Essigsäure gelöst und auf eine Konzentration von 0,1mg/ml bei 4°C eingestellt. Die Rekonstitution der Kollagenmoleküle erfolgt in zwei Prozeßschritten; der pH-Wert-Einstellung auf 7,4 mit doppelt konzentriertem Phosphatpuffer und der Temperaturerhöhung auf 36°C. Nach 3 Stunden besteht die Lösung aus nativ rekonstituierten Fibrillen. Die Probe verbleibt für 10 Minuten in dieser Lösung, danach wird sie mit deionisiertem Wasser gespült.
Die mit Kollagen beschichtete Probe wird als Arbeitselektrode in eine Drei-Elektroden-Anordnung, bestehend aus einer gesättigten Kalomelelektrode als Bezugselektrode und einem Platinblech als Gegenelektrode in einer thermostatierten Elektrolysezelle eingebracht. Als Elektrolytlösung dient eine Stammlösung, die folgendermaßen hergestellt wird: Jeweils 10 ml Stammlösung von CaCl₂ und NH₄H₂PO₄ in den Konzentrationen 33 mM und 20 mM werden verdünnt und gemischt, so daß 200 ml entstehen; 1,67 mM an Calcium- und 1,0 mM an Phosphationen. Der pH-Wert wird mit verdünnter NH₄OH-Lösung auf 6,4 eingestellt.
Nach Verbindung mit dem Potentiostaten erfolgt die Mineralisierung/Beschichtung mit Calciumphosphatphasen (CPP) über galvanostatische Polarisation unter kathodischem Stromfluß bei -1 mA/cm². Nach 30 Minuten wird die kathodische Polarisation beendet, die Probe aus der Elektrolytlösung herausgeholt und mit deionisiertem Wasser gespült. Die abgeschiedene Schicht erscheint weißlich. Die elektronenmikroskopische Betrachtung zeigt eine Schicht, bestehend aus einem Kollagennetzwerk und kugeligen CP-Clustern. IR-spektroskopische Untersuchungen erbringen den Nachweis, daß die mineralische Phase aus amorphen Calciumphosphat besteht.

### Beispiel 2

Ein Zylinder aus TiAI6V4 wird wie im Beispiel 1 hergestellt. Der Aufbau der Elektrolysezelle und der Elektrolyt für die Calciumphosphatabscheidung sind dem in Beispiel 1 identisch.
Nach Verbindung mit dem Potentiostaten erfolgt die Beschichtung mit CPP über galvanostatische Polarisation unter kathodischem Stromfluß bei -10 mA/cm². Nach 30 Minuten wird die kathodische Polarisation unterbrochen, die Probe aus der Elektrolytlösung herausgeholt und mit deionisiertem Wasser gespült. Auf der TiAl6V4-Oberfläche liegt jetzt eine kristalline CPP, Hydroxylapatit, vor. Die Probe wird jetzt in eine Kollagenlösung getaucht, die der in Beispiel 1 identisch ist. In dieser Lösung verbleibt die mit Hydroxylapatit beschichtetet Probe für 10 Minuten, danach wird sie mit deionisiertem Wasser gespült und wieder in die Elektrolysezelle eingebaut. Nach Verbindung mit dem Potentiostaten erfolgt erneut eine Abscheidung von Hydroxylapatit über galvanostatische Polarisation unter kathodischem Stromfluß bei -10 mA/cm². Nach 20 min wird die Probe herausgenommen und mit deionisiertem Wasser gespült. Die abgeschiedene Schicht erscheint weißlich. Die elektronenmikroskopische Betrachtung zeigt eine geschlossene Schicht, die aus Agglomeraten kleiner Nadeln besteht. Auf dieser Schicht befindet sich ein Netzwerk aus mineralisierten Kollagenfibrillen. IR-spektroskopische- und Röntgenbeugungsuntersuchungen erbringen den Nachweis, daß die mineralische Phase aus Hydroxylapatit besteht. Die charakteristischen Amid-Banden im IR-Spektrum zeigen weiterhin, daß das Kollagen nicht denaturiert vorliegt, vielmehr eine gute Übereinstimmung zwischen der mineralisierten Schicht und einem Spektrum für nativen Knochen besteht.

### Beispiel 3

Ein Zylinder aus TiAI6V4 wird wie im Beispiel 1 hergestellt. Der Aufbau der Elektrolysezelle ist dem in Beispiel 1 identisch.
Eine Kollagenlösung mit nativ assemblierten Kollagenfibrillen wird wie im Beispiel 1 hergestellt. Diese Lösung wird für 15 min bei 5000 g und 4°C zentrifugiert, mit deionisiertem Wasser aufgenommen und durch Schütteln dispergiert. Anschließend wird die Lösung erneut für 15 min bei 5000 g und 4°C zentrifugiert. Das bei der Zentrifugation erhaltene Pellet wird jetzt in den Elektrolyten für die Calciumphosphatabscheidung, beschrieben in Beispiel 1, aufgenommen und durch eine Dispergierer homogenisiert. Diese Lösung dient als Elektrolyt für einen simultan geführten Prozeß der Abscheidung und Mineralisierung von Kollagen. Nach Verbindung mit dem Potentiostaten erfolgt die Mineralisierung über galvanostatische Polarisation unter kathodischem Stromfluß bei -10 mA/cm². Nach 30 Minuten wird die kathodische Polarisation beendet, die Probe aus der Elektrolytlösung herausgeholt und mit deionisiertem Wasser gespült.
Die abgeschiedene Schicht erscheint weißlich. Die elektronenmikroskopische Betrachtung zeigt einen Verbund aus Kollagenfibrillen und CPP. IR-spektroskopische- und Röntgenbeugungsuntersuchungen erbringen den Nachweis, daß die Mineralisierung der Fibrillen hauptsächlich durch die kristalline Phase Hydroxylapatit erfolgt. Partiell ist die leichter lösliche amorphe Calciumphosphatphase anzutreffen. Die charakteristischen Amid-Banden im IR-Spektrum zeigen weiterhin, daß das Kollagen nicht denaturiert vorliegt, vielmehr eine gute Übereinstimmung zwischen der mineralisierten Schicht und einem Spektrum für nativen Knochen besteht.

### Beispiel 4

Ein Zylinder aus TiAI6V4 wird wie im Beispiel 1 hergestellt. Der Aufbau der Elektrolysezelle und der Elektrolyt für die Calciumphosphatabscheidung sind dem in Beispiel 1 identisch.
Nach Verbindung mit dem Potentiostaten erfolgt die Beschichtung mit CPP über galvanostatische Polarisation unter kathodischem Stromfluß bei -10 mA/cm². Nach 30 Minuten wird die kathodische Polarisation unterbrochen, die Probe aus der Elektrolytlösung herausgeholt und mit deionisiertem Wasser gespült. Auf der TiAI6V4-Oberfläche liegt jetzt eine kristalline CPP, Hydroxylapatit, vor. Die Probe wird jetzt in eine Kollagenlösung getaucht, die der in Beispiel 1 identisch ist. In dieser Lösung verbleibt die mit Hydroxylapatit beschichtetet Probe für 10 Minuten, danach wird sie mit deionisiertem Wasser gespült und wieder in die Elektrolysezelle eingebaut. Nach Verbindung mit dem Potentiostaten erfolgt eine partielle Mineralisierung des Kollagen unter kathodischem Stromfluß bei -10 mA/cm² für 15 min. Abschließend wird die Probe mit deionisiertem Wasser gespült. Die abgeschiedene Schicht erscheint weißlich. In einem zweiten Verfahrensschritt erfolgt die Anbindung integrinspezifischer zellselektiver Peptidsequenzen an die immobilisierte Kollagenschicht. Die Anbindung erfolgt kovalent über einen Thiolanker und SMPB (Sulfosuccinimidyl 4-(pmaleimidophenyl)butyrat) an die Phosphatgruppen des Kollagens.
Die elektronenmikroskopische Betrachtung zeigt eine homogene Schicht aus Hydroxylapatitnadeln, auf der ein partiell mineralisiertes Netzwerk aus Kollagenfibrillen vorliegt. Die Aktivität der RGD-Sequenzen wird aus Adhäsions- und Proliferationsexperimenten mit MC3T3-E1-Zellen ersichtlich. Gegenüber vergleichbaren reinen Kollagenschichten zeigen die RGD-beschichteten Oberflächen eine erhöhte Zelladhärenz und eine nach kürzeren Zeiten beginnende Zellproliferation.

Abbildung 1 zeigt die Zellproliferation von MC3T3 Maus Osteoplasten auf Hydroxylapatit und auf der knochenanalogen Kollagen-Hydroxylapatit-Matrix jeweils auf TiAl6V4-Substraten. Die Absorption ist proportional zur Zellzahl (WST-1 Test).

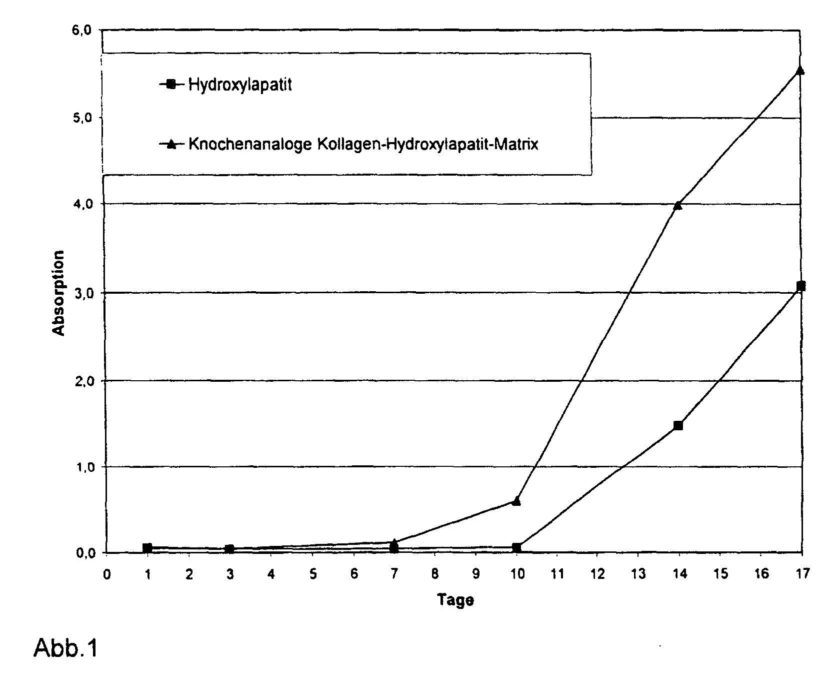

## Patentansprüche

1. Knochenanaloge Beschichtung bestehend aus einem organischen und anorganischen Hauptbestandteil für metallische Implantatmaterialien beliebiger Oberflächengeometrie, **dadurch gekennzeichnet, dass** sie im wesentlichen aus einer mit Calciumphosphat mineralisierten Kollagenmatrix besteht.

2. Beschichtung nach Anspruch 1, **dadurch gekennzeichnet, dass** der Aufbau der mineralisierten Kollagenmatrix schichtförmig ist.

3. Beschichtung nach Anspruch 1 oder 2, **dadurch gekennzeichnet, dass** die
Calciumphosphatphase aus amorphen Calciumphosphat (Ca₉(PO₄)₆ • nH₂O), Hydroxylapatit (Ca₁₀(PO₄)₆(OH)₂), Octacalciumphosphat (Ca₈H₂(PO₄)₆ • 5H₂O) oder Brushit (CaHPO₄ • 2H₂O) besteht.

4. Beschichtung nach einem der Ansprüche 1 bis 3, **dadurch gekennzeichnet, dass** die Calciumphosphatphase mit zusätzlichen Ionen wie Fluorid, Silber, Magnesium oder Carbonat dotiert ist.

5. Beschichtung nach einem der Ansprüche 1 bis 4, **dadurch gekennzeichnet, dass** das Kollagen aus Kollagen Typ I besteht.

6. Beschichtung nach einem der Ansprüche 1 bis 4, **dadurch gekennzeichnet, dass** das Kollagen eine Mischung aus Kollagen der Typen I bis III darstellt.

7. Beschichtung nach einem der Ansprüche 1 bis 6, **dadurch gekennzeichnet, dass** dem Kollagen Gelatine zugesetzt ist.

8. Beschichtung nach einem der Ansprüche 1 bis 7, **dadurch gekennzeichnet, dass** sie bioaktive Substanzen wie Wachstumsfaktoren, Peptidsequenzen, Hormone, Antibiotika oder die Mineralisierung beeinflussende Stoffklassen enthalten.

9. Metallisches Implantat bestehend aus einem Grundkörper und einer von diesem getragenen Außenschicht, **dadurch gekennzeichnet, dass** die Außenschicht aus einer Beschichtung nach einen der Ansprüche 1 bis 8 besteht.

10. Metallisches Implantat nach Anspruch 9, **dadurch gekennzeichnet, dass** es aus Titan oder Titanlegierungen besteht.

11. Verfahren zur elektrochemischen Beschichtung von metallischen Implantatmaterialien beliebiger Oberflächengeometrie mit Kollagen und Calciumphosphatphasen (CPP), **gekennzeichnet durch** folgende Verfahrensschritte:
a) Beschichtung des metallischen Implantatmaterials **durch** Eintauchen in eine Kollagenlösung bei einem pH-Wert kleiner 8 und einer Temperatur zwischen 4 und 40 °C für wenige Minuten.
b) Beschichtung der Kollagen-beschichteten Probe mit CPP in einem elektrochemisch gestütztem Prozess über galvanostatische Polarisation in einer Calciumionen und Phosphationen enthaltenen Elektrolytlösung bei genau definierter Stromdichte und Temperatur.

12. Verfahren nach Anspruch 11, **dadurch gekennzeichnet, dass** ein zusätzlicher Verfahrensschritt b) vor Verfahrensschritt a) vorangestellt wird.

13. Verfahren nach Anspruch 11, **dadurch gekennzeichnet, dass** die Verfahrenschritte a) und b) mehrfach alternierend ablaufen.

14. Verfahren nach Anspruch 11, **dadurch gekennzeichnet, dass** die Verfahrenschritte a) und b) in einem Schritt zusammengefaßt werden, wobei das zu beschichtende metallische Implantatmaterial in einer Calciumionen und Phosphationen enthaltenen Kollagenlösung kathodisch elektrochemisch polarisiert wird.

15. Verfahren nach einem der Ansprüche 11 bis 14, **dadurch gekennzeichnet, dass** während der galvanostatischen Polarisation im Verfahrenschritt b) ein kathodischer Stromfluss von -0.5 bis -30 mA/cm² etwa 30 Minuten lang fließt.

16. Verfahren nach einem der Ansprüche 11 bis 15, **dadurch gekennzeichnet, dass** der Aufbau der mineralisierten Kollagenmatrix schichtförmig erfolgt.

17. Verfahren nach einem der Ansprüche 11 bis 16, **dadurch gekennzeichnet, dass** dem Kollagen Gelatine zugesetzt wird.

## Claims

1. Bone-analogous coating consisting of an organic and inorganic principal constituent for metallic implant materials of any desired surface geometry, **characterised in that** it essentially consists of a collagen matrix mineralised with calcium phosphate.

2. Coating according to Claim 1, **characterised in that** the mineralised collagen matrix has a layered structure.

3. Coating according to Claim 1 or 2, **characterised in that** the calcium phosphate phase consists of amorphous calcium phosphate (Ca₉(PO₄)₅ · nH₂O), hydroxylapatite (Ca₁₀(PO₄)₆(OH)₂), octacalcium phosphate (Ca₈H₂(PO₄)₆ · 5H₂O) or brushite (CaHPO₄ · 2H₂O).

4. Coating according to one of Claims 1 to 3, **characterised in that** the calcium phosphate phase is doped with additional ions, such as fluoride, silver, magnesium or carbonate.

5. Coating according to one of Claims 1 to 4, **characterised in that** the collagen consists of type I collagen.

6. Coating according to one of Claims 1 to 4, **characterised in that** the collagen is a mixture of type I to III collagen.

7. Coating according to one of Claims 1 to 6, **characterised in that** gelatine is added to the collagen.

8. Coating according to one of Claims 1 to 7, **characterised in that** it comprises bioactive substances, such as growth factors, peptide sequences, hormones, antibiotics or substance classes which influence mineralization.

9. Metallic implant consisting of a basic body and an outer layer supported thereby, **characterised in that** the outer layer consists of a coating according to one of Claims 1 to 8.

10. Metallic implant according to Claim 9, **characterised in that** it consists of titanium or titanium alloys.

11. Process for the electrochemical coating of metallic implant materials of any desired surface geometry with collagen and calcium phosphate phases (CPP), **characterised by** the following process steps:
a) coating of the metallic implant material by immersion in a collagen solution at a pH of less than 8 and a temperature between 4 and 40°C for a few minutes,
b) coating of the collagen-coated sample with CPP in an electrochemically supported process via galvanostatic polarisation in an electrolyte solution comprising calcium ions and phosphate ions at a precisely defined current density and temperature.

12. Process according to Claim 11, **characterised in that** an additional process step b) is carried out before process step a).

13. Process according to Claim 11, **characterised in that** process steps a) and b) are carried out alternately a number of times.

14. Process according to Claim 11, **characterised in that** process steps a) and b) are combined in a single step, where the metallic implant material to be coated is subjected to cathodic electrochemical polarisation in a collagen solution comprising calcium ions and phosphate ions.

15. Process according to one of Claims 11 to 14, **characterised in that** a cathodic current flow of from -0.5 to -30 mA/cm² flows for about 30 minutes during the galvanostatic polarisation in process step b).

16. Process according to one of Claims 11 to 15, **characterised in that** the mineralised collagen matrix is built up in layers.

17. Process according to one of Claims 11 to 16, **characterised in that** gelatine is added to the collagen.

## Revendications

1. Revêtement analogue à l'os, constitué d'un constituant principal organique et minéral pour des matériaux d'implant métallique d'une géométrie de surface désirée quelconque, **caractérisé en ce qu'**il est constitué essentiellement d'une matrice de collagène minéralisée par du phosphate de calcium.

2. Revêtement selon la revendication 1, **caractérisé en ce que** la matrice de collagène minéralisée a une structure en couches.

3. Revêtement selon la revendication 1 ou 2, **caractérisé en ce que** la phase de phosphate de calcium est constituée de phosphate de calcium (Ca₉(PO₄)₅ · nH₂O) amorphe, d'hydroxyapatite (Ca₁₀(PO₄)₆(OH)₂), de phosphate d'octacalcium (Ca₈H₂(PO₄)₆ · 5H₂O) ou de brushite (CaHPO₄ · 2H₂O).

4. Revêtement selon l'une des revendications 1 à 3, **caractérisé en ce que** la phase de phosphate de calcium est dopée par des ions supplémentaires, tels que le fluorure, l'argent, le magnésium ou le carbonate.

5. Revêtement selon l'une des revendications **1** à **4, caractérisé en ce que** le collagène est constitué de collagène de type I.

6. Revêtement selon l'une des revendications 1 à 4, **caractérisé en ce que** le collagène est un mélange de collagène de type I à III.

7. Revêtement selon l'une des revendications 1 à 6, **caractérisé en ce que** l'on ajoute de la gélatine au collagène.

8. Revêtement selon l'une des revendications 1 à 7, **caractérisé en ce qu'**il comprend des substances bioactives, telles que des facteurs de croissance, des séquences peptidiques, des hormones, des antibiotiques ou des classes de substances qui influencent la minéralisation.

9. Implant métallique constitué d'un corps de base et d'une couche externe ainsi supportée, **caractérisé en ce que** la couche externe est constituée d'un revêtement selon l'une des revendications 1 à 8.

10. Implant métallique selon la revendication 9, **caractérisé en ce qu'**il est constitué de titane ou d'alliages de titane.

11. Procédé de revêtement électrochimique de matériaux d'implant métallique d'une géométrie de surface désirée quelconque par du collagène et des phases de phosphate de calcium (PPC), **caractérisé par** les étapes de procédé suivantes :
a) le revêtement du matériau d'implant métallique par une immersion dans une solution de collagène à un pH inférieur à 8 et à une température comprise entre 4 et 40°C pendant quelques minutes,
b) le revêtement de l'échantillon revêtu de collagène par des PPC dans un procédé à support électrochimique par l'intermédiaire d'une polarisation intensiostatique dans une solution d'électrolytes comprenant des ions calcium et des ions phosphate à une densité de courant et une température définies de façon précise.

12. Procédé selon la revendication 11, **caractérisé en ce qu'**une étape de procédé supplémentaire b) est réalisée avant l'étape de procédé a).

13. Procédé selon la revendication 11, **caractérisé en ce que** les étapes de procédé a) et b) sont réalisées de manière alternative un certain nombre de fois.

14. Procédé selon la revendication 11, **caractérisé en ce que** les étapes de procédé a) et b) sont associées en une seule étape, dans laquelle le matériau d'implant métallique à revêtir est soumis à une polarisation électrochimique cathodique dans une solution de collagène comprenant des ions calcium et des ions phosphate.

15. Procédé selon l'une des revendications **11** à **14, caractérisé en ce qu'**une circulation de courant cathodique allant de -0.5 à -30 mA/cm² passe pendant environ 30 minutes pendant la polarisation intensiostatique dans l'étape de procédé b).

16. Procédé selon l'une des revendications 11 à 15, **caractérisé en ce que** la matrice de collagène minéralisée est construite en couches.

17. Procédé selon l'une des revendications 11 à 16, **caractérisé en ce que** l'on ajoute de la gélatine au collagène.
